(19) Europäisches Patentamt
European Patent Office
Office européen des brevets

(11) **EP 0 594 014 B1**

(12) **EUROPÄISCHE PATENTSCHRIFT**

(45) Veröffentlichungstag und Bekanntmachung des Hinweises auf die Patenterteilung:
**10.04.1996 Patentblatt 1996/15**

(21) Anmeldenummer: **93116386.9**

(22) Anmeldetag: **09.10.1993**

(51) Int. Cl.$^6$: **C01B 6/24**, C07B 35/02,
C07C 29/143, C07C 29/17,
C07C 29/147, C07C 31/125,
C07C 33/12, C07C 33/03,
C07C 33/24

(54) **Verfahren zur Herstellung von Halogen-Magnesium-Aluminiumhydridhalogeniden**

Process for the production of magnesium-halide-aluminium-hydridohalide

Méthode pour la production d'une halogénure de magnésium-hydridohalogénure d'aluminium.

(84) Benannte Vertragsstaaten:
**BE DE FR GB IT NL**

(30) Priorität: **20.10.1992 DE 4235334**

(43) Veröffentlichungstag der Anmeldung:
**27.04.1994 Patentblatt 1994/17**

(73) Patentinhaber: **Th. Goldschmidt AG**
**D-45127 Essen (DE)**

(72) Erfinder: **Knott, Wilfried, Dr.**
**D-45141 Essen (DE)**

(56) Entgegenhaltungen:
**US-A- 4 832 934**

• **INORGANIC CHEMISTRY Bd. 16, Nr. 11 , 1977 , EASTON US Seiten 2941 - 2944 E.C.ASHBY, A.B.GOEL 'Preparation of HMgX compounds and their reactions with alane and borane in tetrahydrofuran'**
• **J.Serb.Chem.Soc. 54(11),1989, pages 579-587**

**Beschreibung**

Die Erfindung betrifft ein Verfahren zur Herstellung von Halogen-Magnesium-Aluminiumhydridohalogeniden der allgemeinen Formel

$$[Mg_2X_3(Ether)_y]^+[AlH_{4-n}X_n]^-$$

wobei

X    Chlor, Brom oder Jod,
y    eine Zahl von 0 bis 6,
n    eine Zahl von 1 bis 3 und

Ether ein aliphatischer oder cycloaliphatischer Ether mit 2 bis 30 Kohlenstoffatomen, ausgenommen tert.-Butylether, mit einem Dipolmoment > 0,5 Debye und einem Schmelzpunkt < 0°C ist.

Reduktionen mit komplexen Metallhydriden sind Standardreaktionen der organischen Chemie. Zu diesem Stand der Technik wird auf N.G. Gaylord, Reduction with Complex Metal Hydrides, Interscience Publishers, Inc., New York, 1956, verwiesen. Dabei wird am häufigsten das etherlösliche $LiAlH_4$ verwendet. Gelänge es, das relativ teuere Lithium durch das billigere Mg zu ersetzen, könnte man die bei der Reduzierung anfallenden Kosten erheblich senken. Es hat deshalb nicht an Versuchen gefehlt, $Mg(AlH_4)_2$ zu synthetisieren. Man findet jedoch in der Literatur widersprüchliche Angaben zur Herstellung und den Eigenschaften von $Mg(AlH_4)_2$.

Ein in Ether lösliches $Mg(AlH_4)_2$ wurde erstmals von Wiberg und Bauer in der Zeitschrift für Naturforschung 5B, 397 (1950) und 7B, 131 (1952) sowie in der DE-PS 845 338 beschrieben. Die Herstellung sollte nach folgenden Verfahren gelingen:

$$4\ MgH_2 + 2\ AlCl_3 \rightarrow Mg(AlH_4)_2 + 3\ MgCl_2 \tag{1}$$

$$MgH_2 + 2\ AlH_3 \rightarrow Mg(AlH_4)_2 \tag{2}$$

$$2\ LiAlH_4 + MgBr_2 \rightarrow Mg(AlH_4)_2 + 2\ LiBr \tag{3}$$

Das für die Reaktion (1) und (2) benötigte $MgH_2$ wurde durch Thermolyse von Grignard- bzw. Diorganomagnesiumverbindungen gewonnen. Dabei fallen neben Magnesiumhalogenid noch Alkene als unerwünschte Nebenprodukte an. Das Verfahren (3) hat den Nachteil der Verwendung von relativ teurem $LiAlH_4$. Dabei hat Ashby später festgestellt, daß diese Reaktion (3) in Tetrahydrofuran nicht zu $Mg(AlH_4)_2$, sondern zu $BrMgAlH_4$ führt, welches auch aus $HMgBr$ und $AlH_3$ zugänglich ist (Ashby et al., Inorg. Chem. 9, 325 (1970) und Inorg. Chem. 16, 941 (1977)).

Nach Hertwig (DE-PS 921 986) entsteht bei der Umsetzung von Grignardverbindungen mit $AlX_3$ und Wasserstoff $ClMgAlH_4$, welches durch Thermolyse in Magnesiumalanat überführt werden kann:

$$4\ RMgX + AlX_3 + 4\ H_2 \rightarrow XMg(AlH_4) + 3\ MgX_2 + 2\ RH \tag{4}$$

$$XMg(AlH_4) \rightarrow Mg(AlH_4)_2 + MgX_2 \tag{5}$$

Nach der GB-PS 785 348 führt die Umsetzung einer Mischung von $MgH_2$ und Aluminium zu $Mg(AlH_4)_2$.

Die US-PS 3 387 948 betrifft die Umsetzung

$$MgH_2 + 3\ H_2 + 2\ Al \rightarrow Mg(AlH_4)_2 \tag{6}$$

und soll unter drastischen Reaktionsbedingungen ablaufen.

Alle diese Verfahren genügen aber technischen Ansprüchen nicht.

Das US-Patent 4 832 934 greift erneut die Herstellung von $[Mg_2X_3(Ether)_y]^+[AlH_4]^-$ auf, wobei X Halogen, Ether ein cyclischer oder aliphatischer Ether und y eine Zahl von 0 bis 6 ist. Es wird in dieser Patentschrift in Spalte 1, Zeile 65 bis Spalte 2, Zeile 2, ausgeführt, daß die Substitution des teuren Lithiumalanates gegen das billigere, lösliche Halogen-Magnesium-Alanat noch immer wünschenswert erscheint. Die Realisierung dieses Konzeptes sei aber immer daran gescheitert, daß bisher eine effiziente Synthese für das billige Ausgangsprodukt $MgH_2$ gefehlt habe.

Das US-Patent 4 832 934 lehrt nun, daß das hochaktive $MgH_2$, welches gemäß einem Verfahren der EP-PS 0 003 564 hergestellt ist, sich in hervorragender Weise zur Erzeugung von Halogen-Magnesium-Alanaten aus Aluminiumhalogeniden eignet.

Das Verfahren der EP-PS 0 003 564 zur Herstellung von Hydriden des Magnesiums mit Hilfe von Wasserstoff und Übergangsmetallen als Katalysatoren ist dadurch gekennzeichnet, daß man Magnesium in Gegenwart eines Katalysators, bestehend aus einem Halogenid eines Metalles der IV. bis VIII. Nebengruppe des periodischen Systems und einer metallorganischen Verbindung bzw. eines Magnesiumhydrids sowie gegebenenfalls in Gegenwart eines polycyclischen Aromaten oder eines tertiären Amins sowie gegebenenfalls in Gegenwart eines Magnesiumhalogenides $MgX_2$ mit Wasserstoff umsetzt. Vorzugsweise erfolgt die Umsetzung in Tetrahydrofuran bei Drucken von 1 bis 300 bar und bei Temperaturen von 0 bis 200°C, wobei das Verhältnis von Magnesium Übergangsmetall wie $10^4$ bis 10 : 1 und das Verhältnis von Übergangsmetall : magnesiumorganischer Verbindung bzw. Magnesiumhydrid wie 0,1 : 1 bis 10 : 1 betragen soll. Als Übergangsmetallhalogenide werden insbesondere Chrom-, Titan- und Eisenhalogenide eingesetzt. Als magnesiumorganische Verbindung wird Magnesiumanthracen verwendet. Als polycyclische Aromaten werden Anthracen, Tetracen oder Benzanthracen gewählt. Als tertiäre Amine dienen solche der Formel $NR_3$ mit R = Alkyl- oder Cycloalkylgruppen.

Hiervon ausgehend beschreibt die US-PS 4 832 934 die Darstellung der Halogen-Magnesium-Alanate unter Verwendung dieses speziellen, durch komplexe Katalysatorgemische zugänglichen Magnesiumhydrids, indem dieses hochdisperse Magnesiumhydrid mit Aluminiumhalogeniden in einem Lösungsmittel umgesetzt wird. Die Verwendung dieses komplexen Katalysators wird als zwingend notwendig herausgestellt. In Spalte 3, Zeilen 27 bis 34, wird ausdrücklich gesagt, daß die Herstellung des $[Mg_2X_3(Ether)_y]^+[AlH_4]^-$ nicht gelingt, wenn man Magnesiumhydrid verwendet, welches aus den Elementen bei hoher Temperatur hergestellt worden ist. In einem Vergleichsversuch (Spalte 5, Comparative Example) wird käufliches, bei hohen Temperaturen aus den Elementen hergestelltes Magnesiumhydrid über 2 Stunden in einer Glaskugelmühle vorvermahlen und dann mit $AlCl_3$, Tetrahydrofuran und Benzoesäureethylester versetzt. Das Gemisch war inaktiv, eine Reduktion des Benzoesäureesters zu Benzylalkohol konnte nicht festgestellt werden. Es hatte sich somit kein $[Mg_2X_3(Ether)_y]^+[AlH_4]^-$ als reduzierendes Agens gebildet.

Gemäß Bogdanovic (J. Serb. Chem. Soc. 54 (11), pp 579-587 (1989)) wird angenommen, daß man, ausgehend von HMgCl und $AlCl_3$, verschiedene Chlormagnesiumalanate erhält, die in der erhaltenen Mischung im Gleichgewicht vorliegen. Hierzu ist ein Hydridomagnesiumchlorid einzusetzen, welches erhalten wird durch Hydrierung von Magnesium in THF-Lösungen von Magnesiumchlorid in Gegenwart eines Magnesium-Anthracen/$CrCl_3$.

Die vorliegende Erfindung befaßt sich mit dem technischen Problem, dieses billige, nach US-PS 4 832 934 inaktive Magnesiumhydrid für die Herstellung komplexer Magnesium-Aluminium-Hydridverbindungen einsetzbar zu machen, so daß man auf die Verwendung schwierig zu handhabender und teurer komplexer Katalysatoren entsprechend der EP-PS 0 003 564 verzichten kann. Dies erscheint insbesondere zur Vermeidung produktkontaminierender Verunreinigungen unter dem Gesichtspunkt einer technischen Syntheseplanung wünschenswert.

Überraschenderweise wurde nun gefunden, daß dieses Problem gelöst wird, indem man Verbindungen der allgemeinen Formel

$$[Mg_2X_3(Ether)_y]^+[AlH_{4-n}X_n]^-$$

wobei

X     Chlor, Brom oder Jod,
y     eine Zahl von 0 bis 6,
n     eine Zahl von 1 bis 3 und

Ether ein aliphatischer oder cycloaliphatischer Ether mit 2 bis 30 Kohlenstoffatomen, ausgenommen tert.-Butylether, mit einem Dipolmonent > 0,5 Debye und einem Schmelzpunkt < 0°C ist, dadurch herstellt, daß man das Aluminiumhalogenid $AlX_3$ mit Magnesiumhydrid $MgH_2$ unter ständiger Aufmahlung in einem organischen Lösungsmittel umsetzt.

Entscheidend ist somit die aktivierende Aufmahlung während der gesamten Reaktionszeit und nicht, wie in der US-PS 4 832 934 beschrieben, vor der Reaktion. Geeignete Mahlvorrichtungen sind Schwingmühlen, Attritormühlen, Ringspaltrührwerkskugelmühlen, Wheelermühlen, Jet-O-Mizer, Eagle-Mühlen, Fließbettstrahlmühlen, Perlmühlen nach Art der Dynomill bzw. Supermill.

Vorzugsweise wird ein Magnesiumhydrid verwendet, welches entsprechend dem Verfahren der DE-OS 40 39 278 hergestellt worden ist. Bevorzugt ist somit ein Verfahren mit dem Kennzeichen, daß man bei der Umsetzung $MgH_2$ verwendet, welches aus den Elementen hergestellt ist, wobei man dem zu hydrierenden Magnesium als Katalysator von Beginn der Reaktion an Magnesiumhydrid einer Teilchengröße von $\leq$ 400 µm in einer Menge von mindestens 1,2 Gew.-%, bezogen auf zu hydrierendes Magnesium, zusetzt, und die Hydrierung bei einer Temperatur von $\geq$ 250°C und einem Druck von 0,5 bis 5 MPa unter ständigem Rühren des Reaktionsgutes durchführt.

Für das erfindungsgemäße Verfahren zur Herstellung von

$$[Mg_2X_3(Ether)_y]^+[AlH_{4-n}X_n]^-$$

wird vorzugsweise Tetrahydrofuran, Diethylether, Dimethoxyethan (Glyme), Di-n-butylether oder 1,3-Dioxolan verwendet. Tetrahydrofuran ist jedoch besonders bevorzugt.

| Erfindungsgemäßer Ether | Dipolmoment in Debye | Schmelzpunkt in °C |
|---|---|---|
| Tetrahydrofuran | 1,63 | - 108 |
| Diethylether | 1,18 | - 116 |
| Dimethoxyethan | 1,81 | - 58 |
| Di-n-butylether | 1,18 | - 98 |
| 1,3-Dioxolan | 1,47 | - 95 |

Die Reaktion kann bei Zimmertemperatur ablaufen, sie wird jedoch vorzugsweise bei einer Temperatur von -80° bis +150°C, insbesondere bei -10° bis +60°C durchgeführt.

Das erfindungsgemäße Verfahren und insbesondere die im Patentanspruch 2 beschriebene bevorzugte Variante ermöglicht eine Synthese von Halogen-Magnesium-Aluminiumhydridohalogeniden aus den billigen Rohstoffen Magnesium, Wasserstoff und Aluminiumhalogenid, ohne daß kontaminierende Katalysatoren involviert sind.

Die Halogen-Magnesium-Aluminiumhydridohalogenide können direkt in Lösung oder nach Entfernen des Lösungsmittels in fester Form zu Reduktionszwecken eingesetzt werden. Das als Nebenprodukt anfallende $MgCl_2$ stört bei den Folgereaktionen nicht und kann gegebenenfalls entfernt werden.

Die Halogen-Magnesium-Aluminiumhydridohalogenide sind gute Reduktionsmittel für eine Vielzahl funktioneller Gruppen in organischen Verbindungen, wie Aldehyde, Ester, Ketone, Alkylhalogenide, Carbonsäuren und deren Anhydride, wobei die Reduktion in sehr guten Ausbeuten erfolgt. Ein Wechsel des Lösungsmittels für die Reduktionen ist möglich.

Die Verbindungen verbinden, da sie nicht pyrophor sind, äußerst sichere Handhabbarkeit mit hohem Reduktionsvermögen bei niedrigerem Preis als vergleichbare Metallhydride und sind daher wesentlich vorteilhafter zu verwenden als z.B. Lithiumalanat.

In den folgenden Beispielen wird die Herstellung der erfindungsgemäßen Verbindungen durch das erfindungsgemäße Verfahren gezeigt und ihr Reduktionsvermögen demonstriert. Alle Versuche wurden unter Argon als Schutzgas durchgeführt.

Beispiel 1

19,5 g (0,67 Mol) Magnesiumhydrid (Hydridgehalt 91 %) werden in 400 g Tetrahydrofuran suspendiert und durch Aufmahlung in einer Glaskugelmühle aktiviert. 40,0 g (0,3 Mol) Aluminiumchlorid, gelöst in 160 g Tetrahydrofuran, werden langsam hinzugetropft, wobei eine leichte Wärmeentwicklung feststellbar ist. Nach beendetem Zutropfen wird der Reaktionsansatz unter weiterer, ständiger Aufmahlung auf Rückflußtemperatur erhitzt. Reaktionsbegleitend werden mittels einer Spritze Proben aus dem Reaktor entnommen, zentrifugiert, der klare Überstand hydrolysiert und hieraus der Säurewert bestimmt. Nach 4 Stunden reagiert der klare Überstand alkalisch. Eine gasvolumetrische Wasserstoffbestimmung belegt einen Wasserstoffgehalt von 1,5 mMol H/g Lösung. Für Reduktionsversuche wird der gesamte Ansatz zentrifugiert und die klare Hydridlösung weiterverwendet.

Beispiel 2

22,4 g (11,2 mMol) der in Beispiel 1 hergestellten Chlormagnesium-Aluminiumhydridochlorid-Lösung werden in einem Schlenkgefäß unter Magnetrührung vorgelegt und dann werden 1,9 ml (15 mMol) Benzoesäuremethylester langsam zupipettiert. Nach Abklingen der kräftigen Exothermie läßt man für eine Stunde nachrühren und zersetzt dann den Reaktionsansatz, indem man die Reaktionsmischung in eisgekühlte wäßrige HCl gießt. Nach Phasentrennung und wiederholtem Ausethern der Wasserphase werden die vereinigten etherischen Extrakte über $Na_2SO_4$ getrocknet. Nach Abziehen des Lösungsmittels wird der Rückstand $^1$H-NMR-spektroskopisch analysiert. Ausbeute: 85 % Benzylalkohol.

Beispiel 3

Der Vorgehensweise des Beispiels 2 folgend, werden 22,4 g (11,2 mMol) Chlor-Magnesium-Aluminiumhydridochlorid-Lösung mit 1,81 g (15 mMol) Acetophenon umgesetzt. Nach Aufarbeitung belegt die $^1$H-NMR-Spektroskopie eine 95%ige Ausbeute an 1-Phenylethanol.

Beispiel 4

Den Beispielen 2 und 3 entsprechend, werden 3,18 g (30 mMol) Benzaldehyd mit 44,8 g (22,4 mMol) Chlor-Magnesium-Aluminiumhydridochlorid-Lösung umgesetzt. Die spektroskopisch bestimmte Ausbeute an Benzylalkohol beträgt 97 %.

Beispiel 5

Analog den vorangegangenen Beispielen werden 2,77 g (15 mMol) 10-Undecensäure mit 22,4 g (11,2 mMol) Chlor-Magnesium-Aluminiumhydridochlorid-Lösung zur Reaktion gebracht. Nach üblicher Aufarbeitung wird 10-Undecylalkohol in 57 % Ausbeute nachgewiesen ($^1$H-NMR).

Beispiel 6

Gemäß den vorangegangenen Beispielen werden 2,46 g (15 mMol) 5-Norbornen-2,3-dicarbonsäureanhydrid mit 39,2 g (19,6 mMol) der im Beispiel 1 dargestellten Chlor-Magnesium-Aluminiumhydridochlorid-Lösung umgesetzt. Die Ausbeute an 2,3-Bis-(hydroxymethyl)-norbornen-(5) beträgt 85 % ($^1$H-NMR).

Beispiel 7

2,10 g (30 mMol) Crotonaldehyd werden mit 35 ml (10,65 mMol) einer gemäß Beispiel 1 hergestellten Chlor-Magnesium-Aluminiumhydridochlorid-Lösung zur Reaktion gebracht. Der hierbei gebildete Crotylalkohol entsteht in einer Ausbeute von 88 % ($^1$H-NMR).

Beispiel 8

Den anderen Beispielen folgend werden 3,00 g (30 mMol) Methylisobutylketon mit 30 g (15 mMol) der in Beispiel 1 hergestellten Chlor-Magnesium-Aluminiumhydridochlorid-Lösung umgesetzt. Die $^1$H-NMR-Spektroskopie belegt eine 82 %ige Ausbeute an 4-Methylpentan-(2)-ol.

Beispiel 9

2,73 g (15 mMol) Benzophenon werden mit 30 ml (8,13 mMol) einer gemäß Beispiel 1 hergestellten Chlor-Magnesium-Aluminiumhydridochlorid-Lösung umgesetzt. Nach Aufarbeitung liegt die $^1$H-NMR-spektroskopisch bestimmte Ausbeute an Diphenylmethanol bei 94 %.

Beispiel 10

9,2 g (0,32 Mol) Magnesiumhydrid (Hydridgehalt 91 %) werden mit 21,0 g (0,16 Mol) Aluminiumchlorid in einer Glaskugelmühle durch Aufmahlung aktiviert. 450 ml 1,2-Dimethoxyethan werden hinzugehebert, wobei eine leichte Wärmeentwicklung feststellbar ist. Nach beendeter Zugabe wird der Reaktionsansatz unter weiterer, ständiger Aufmahlung auf Rückflußtemperatur erhitzt. Die Viskosität des Reaktionsgemisches steigt nach Erreichen der Siedetemperatur kräftig an. Nach 2,5 Stunden wir die Reaktionsmischung heiß in inertisierte Zentrifugengläser überführt. Nach Zentrifugieren ergibt die gasvolumetrische Wasserstoffbestimmung der erhaltenen, klaren Hydridlösung einen Gehalt von 0,33 mMol $H^-$/g Lösung.

Beispiel 11

185 g (20,35 mMol) der in Beispiel 10 hergestellten Chlormagnesium-Aluminiumhydridochlorid-Lösung werden in einem Zweihalskolben unter Magnetrührung vorgelegt, und dann werden 2,44 g (25,4 mMol) -Angelicalacton (98 %ig) langsam bei 80°C hinzugetropft. Nach beendeter Zugabe wird 1 Stunde bei 80°C nachgerührt, dann wird die Reaktionsmischung in eisgekühlte, wäßrige NaCl-Lösung gegossen und mit verdünnter HCl neutralisiert. Nach Phasentrennung und wiederholtem Ausethern der Wasserphase werden die vereinigten etherischen Extrakte über $Na_2SO_4$

getrocknet. Nach Abziehen des Lösungsmittels wird der Rückstand $^1$H-NMR-spektroskopisch analysiert. Ausbeute: 75 % Pentandiol-(1,4).

Beispiel 12 (nicht erfindungsgemäß)

13,0 g (0,49 Mol) Magnesiumhydrid (Hydridgehalt 91 %) und 20,0 g (0,15 Mol) Aluminiumchlorid werden unter Aufmahlung in einer Glaskugelmühle vorgelegt. 450 ml 1,4-Dioxan, welches ein Dipolmoment von 0 Debye aufweist, werden hinzugehebert und die Reaktionsmischung unter weiterer, ständiger Aufmahlung auf Rückflußtemperatur erhitzt. Reaktionsbegleitend werden mittels einer Spritze Proben aus dem Reaktor entnommen, zentrifugiert und aus dem klaren Überstand jeweils gasvolumetrisch der Wasserstoffgehalt bestimmt. Nach 3 Stunden Reaktionszeit wird der Versuch abgebrochen, da in der Dioxanphase das erfindungsgemäße Hydrid nicht nachweisbar ist.

Beispiel 13 (nicht erfindungsgemäß)

10,7 g (0,36 Mol) Magnesiumhydrid (Hydridgehalt 90 %) und 24,3 g (0,18 Mol) Aluminiumchlorid werden in einer Glaskugelmühle durch Aufmahlung aktiviert. 450 ml Methyl-tert.-butylether werden auf -70°C vorgekühlt und dann hinzugehebert, wobei eine exotherme Reaktion feststellbar ist. Die Viskosität des Reaktionsansatzes nimmt zu und der Ansatz nimmt eine organe Färbung an. Nach vollständiger Zugabe wird der Ansatz unter weiterer, ständiger Aufmahlung auf Rückflußtemperatur erhitzt. Reaktionsbegleitend werden mittels einer Spritze Proben entnommen, zentrifugiert und der klare Überstand hydrolysiert. Die Wasserstoffbestimmung belegt, daß in der klaren Methyl-tert.-butylether-Phase kein hydridischer Wasserstoff vorliegt.

**Patentansprüche**

1. Verfahren zur Herstellung von Halogen-Magnesium-Aluminiumhydridohalogeniden der allgemeinen Formel

$$[Mg_2X_3(Ether)_y]^+[AlH_{4-n}X_n]^-$$

wobei

X     Chlor, Brom oder Jod,
y     eine Zahl von 0 bis 6,
n     eine Zahl von 1 bis 3 und

Ether ein aliphatischer oder cycloaliphatischer Ether mit 2 bis 30 Kohlenstoffatomen, ausgenommen tert.-Butylether, mit einem Dipolmoment > 0,5 Debye und einem Schmelzpunkt < 0°C ist, dadurch gekennzeichnet, daß man Aluminiumhalogenid $AlX_3$ mit Magnesiumhydrid $MgH_2$ unter ständiger Aufmahlung in dem oben definierten Ether umsetzt.

2. Verfahren nach Anspruch 1, dadurch gekennzeichnet, daß man bei der Umsetzung $MgH_2$ verwendet, welches aus den Elementen hergestellt ist, wobei man dem zu hydrierenden Magnesium als Katalysator von Beginn der Reaktion an Magnesiumhydrid einer Teilchengröße von $\leq$ 400 µm in einer Menge von mindestens 1,2 Gew.-%, bezogen auf zu hydrierendes Magnesium, zusetzt, und die Hydrierung bei einer Temperatur von $\geq$ 250°C und einem Druck von 0,5 bis 5 MPa unter ständigem Rühren des Reaktionsgutes durchführt.

3. Verfahren nach Anspruch 1 oder 2, dadurch gekennzeichnet, daß man als Ether Tetrahydrofuran verwendet.

4. Verfahren nach den Ansprüchen 1 bis 3, dadurch gekennzeichnet, daß man die Umsetzung bei einer Temperatur von -80° bis +150°C, insbesondere bei -10° bis + 60°C durchführt.

**Claims**

1. Process for the preparation of halogen-magnesium-aluminium hydridohalides of the general formula

$$[Mg_2X_3(ether)_y]^+[AlH_{4-n}X_n]^-$$

in which

X     is chlorine, bromine or iodine,

y     is a number from 0 to 6,

n     is a number from 1 to 3, and

the ether is an aliphatic or a cycloaliphatic ether having 2 to 30 carbon atoms, a dipole moment of >0.5 Debye and a melting point of <0°C, with the exception of tert-butyl ether, characterized in that aluminium halide $AlX_3$ is reacted with magnesium hydride $MgH_2$ in the above-defined ether with continuous grinding.

2.    Process according to Claim 1, characterized in that the $MgH_2$ used in the reaction has been prepared from the elements by adding to the magnesium to be hydrogenated, as catalyst from the beginning of the reaction, magnesium hydride with a particle size of $\leq$ 400 $\mu$m in a quantity of at least 1.2 % by weight, based on the magnesium to be hydrogenated, and carrying out the hydrogenation at a temperature of $\geq$ 250°C and at a pressure of from 0.5 to 5 MPa with continuous stirring of the reaction material.

3.    Process according to Claim 1 or 2, characterized in that the ether used is tetrahydrofuran.

4.    Process according to Claims 1 to 3, characterized in that the reaction is carried out at a temperature of from -80 to +150°C, in particular at from -10 to +60°C.

## Revendications

1.    Procédé de préparation d'halogénure de magnésium-hydrurohalogénures d'aluminium répondant à la formule générale :

$$[Mg_2X_3(\text{éther})_y]^+[AlH_{4-n}X_n]^-$$

où

X     est le chlore, le brome ou l'iode,

y     est un nombre de 0 à 6,

n     est un nombre de 1 à 3, et

éther signifie un éther aliphatique ou cycloaliphatique ayant de 2 à 30 atomes de carbone, à l'exception de l'éther tert-butylique, ayant un moment dipolaire > 0,5 Debye et un point de fusion < 0°C, caractérisé en ce qu'on fait réagir un halogénure d'aluminium $AlX_3$ avec de l'hydrure de magnésium $MgH_2$ avec broyage constant dans l'éther défini ci-dessus.

2.    Procédé selon la revendication 1, caractérisé en ce qu'on utilise, pour la réaction, du $MgH_2$ qui a été préparé à partir des éléments et, dès le début de la reaction, on ajoute, comme catalyseur au magnésium à hydrogéner, de l'hydrure de magnésium ayant des particules d'une dimension $\leq$ 400 $\mu$m, en une quantité d'au moins 1,2 % en poids par rapport au magnésium à hydrogéner, et on effectue l'hydrogénation à une température $\geq$ 250°C et à une pression de 0,5 à 5 MPa, avec agitation constante du produit de réaction.

3.    Procédé selon la revendication 1 ou 2, caractérisé en ce qu'on utilise du tétrahydrofuranne comme éther.

4.    Procédé selon les revendications 1 à 3, caractérisé en ce qu'on effectue la réaction à une température de -80°C à +150°C, en particulier de -10°C à +60°C.